# EUROPEAN PATENT APPLICATION

(11) **EP 4 606 367 A2**
(43) Date of publication of application: **27.08.2025**
(21) Application number: 25159363.8
(22) Date of filing: 21.02.2025
(51) Int. Cl.: A61H 9/00

(54) **HOT AND COLD THERAPY SYSTEM**

(30) Priority: 23.02.2024 US 202463557287 P; 12.02.2025 US 202519052047
(71) Applicant: Kohler Co., Kohler, WI 53044 (US)
(72) Inventor: Kwacz, Jason, Kohler, 53044 (US); Revelis, Katerina, Kohler, 53044 (US); Smies, Ariana, Kohler, 53044 (US); Lee, Dongha, Kohler, 53044 (US); Palmer, Douglas, Kohler, 53044 (US)
(74) Representative: Barker Brettell LLP

(57) **Abstract**

A therapy system includes a first therapy assembly configured to provide a hot therapy experience and a second therapy assembly configured to provide a cold therapy experience. The first therapy assembly includes a plurality of walls defining an interior chamber, a door configured to permit selective access to the interior chamber, and a sauna element fluidly coupled with the interior chamber and configured to provide at least one of heated air or steam to the interior chamber. The second therapy assembly includes a bathtub defining a bathing volume structured to receive and hold a fluid. The bathtub is positioned relative to the interior chamber of the first therapy assembly such that the bathing volume is accessible from the interior chamber of the first therapy assembly.

## Description

### CROSS-REFERENCE TO RELATED PATENT APPLICATION

This application claims the benefit of and priority to U.S. Provisional Patent Application No. 63/557,287, filed February 23, 2024, and U.S. Patent Application No. 19/052,047, filed February 12, 2025, which are incorporated herein by reference in their entirety.

### BACKGROUND

The present disclosure relates generally to therapy systems, such as saunas and cold plunge tubs. More specifically, the present disclosure relates to a transition between an experience provided by the sauna and an experience provided by the cold plunge tubs.

### SUMMARY

One embodiment of the present disclosure relates to a therapy system. The therapy systemincludes a first therapy assembly configured to provide a hot therapy experience and a second therapy assembly configured to provide a cold therapy experience. The first therapy assembly includes a plurality of walls defining an interior chamber, a door configured to permit selective access to the interior chamber, and a sauna element fluidly coupled with the interior chamber and configured to provide at least one of heated air or steam to the interior chamber. The second therapy assembly includes a bathtub defining a bathing volume structured to receive and hold a fluid. The bathtub is positioned relative to the interior chamber of the first therapy assembly such that the bathing volume is accessible from the interior chamber of the first therapy assembly.

Another embodiment of the present disclosure relates to a therapy system. The therapy system includes a frame, a platform supported by the frame and configured to support a user, a first spray assembly positioned vertically above the platform, the first spray assembly including a plurality of first spray heads configured to direct a flow of fluid in a direction towards the platform, and a second spray assembly positioned vertically below the platform, the second spray assembly including a plurality of second spray heads configured to direct a flow of fluid in a direction towards the platform.

Still another embodiment relates to a therapy system. The therapy system includes a hot therapy assembly configured to provide a hot therapy experience and acold therapy assembly configured to provide a cold therapy experience. The hot therapy assembly includes a plurality of walls defining an interior chamber and a sauna element fluidly coupled with the interior chamber and configured to provide at least one of heated air or steam to the interior chamber. The cold therapy assembly includes a bathtub defining a bathing volume structured to receive and hold a fluid. The bathtub is positioned below the hot therapy assembly such that at least a portion of the bathtub extends within the interior chamber and the bathing volume is accessible from the interior chamber of the hot therapy assembly.

### BRIEF DESCRIPTION OF THE FIGURES

The disclosure will become more fully understood from the following detailed description, taken in conjunction with the accompanying figures, wherein like reference numerals refer to like elements, in which:
FIG. 1 is a front perspective view of a therapy system in a covered configuration, according to an embodiment;
FIG. 2 is a front perspective view of the therapy system of FIG. 1 in an uncovered configuration;
FIG. 3 is a side perspective view of the therapy system of FIG. 1 in the covered configuration;
FIG. 4 is a front perspective view of a therapy system, according to another embodiment;
FIG. 5 is a block diagram of the therapy system of FIG. 1 and the therapy system of FIG. 4;
FIG. 6 is a block diagram of a control system that can be used with the therapy system of FIG. 1 and the therapy system of FIG. 4;
FIG. 7 is a front perspective view of a therapy system, according to another embodiment;
FIG. 8 is a side view of the therapy system of FIG. 7;
FIG. 9 is a front perspective view of a therapy system, according to another embodiment;
FIG. 10 is a side perspective view of a therapy system, according to another embodiment; and
FIG. 11 is a block diagram of a control system that can be used with the therapy system of FIG. 7, the therapy system of FIG. 9, and the therapy system of FIG. 10.

### DETAILED DESCRIPTION

Before turning to the figures, which illustrate certain exemplary embodiments in detail, it should be understood that the present disclosure is not limited to the details or methodology set forth in the description or illustrated in the figures. It should also be understood that the terminology used herein is for the purpose of description only and should not be regarded as limiting.

Referring generally to the figures, a therapy system is shown that produces a hot therapy experience and a cold therapy experience. The therapy system facilitates a transition between the hot and cold experiences. The therapy system provides an all-in-one cold plunge and sauna, allowing quick and easy transition between hold and cold therapy.

In at least one embodiment, the therapy system includes a hot therapy system configured to provide a hot therapy experience and a cold therapy system configured to provide a cold therapy experience. The hot therapy system defines an interior chamber and includes a sauna element fluidly coupled with the interior chamber. The sauna element can provide the hot therapy experience within the interior chamber by providing at least one of heated air or generated steam to the interior chamber. The cold therapy system includes a bathtub structured to hold liquid such a cold water or ice water. A user can submerge themselves in the ice water held by the bathtub to experience the cold therapy experience. A support structure such as a platform can be removably coupled with and supported by an upper edge of the bathtub. In a covered configuration, the support structure covers the bathtub such that access to the ice water held by the bathtub is prevented (e.g., such that a user cannot climb into the bathtub or submerge themselves in the ice water). The cold therapy system is positioned below the hot therapy system such that at least a portion of the bathtub extends within the interior chamber. As such, in the covered configuration, a user can rest (e.g., sit, lay, stand, etc.) on the support structure to experience the hot therapy experience provided within the interior chamber. In an uncovered configuration, the support structure is removed to permit access to the ice water held by the bathtub (e.g., such that a user can climb into the bathtub or submerge themselves in the ice water). Transitioning the support structure between the covered configuration and the uncovered configuration facilitates transitioning the therapy system between the hot therapy experience and the cold therapy experience.

In at least another embodiment, a therapy system is configured to direct a flow of fluid at a user from various different angles and sources. The therapy system can include a platform supported by a frame and configured to support a user. The therapy system can include a first spray assembly supported by the frame and positioned vertically above the platform. The first spray system can include a plurality of spray heads to direct a flow of fluid in a direction towards the platform (e.g., towards a user on the platform) and along a length of the platform (e.g., along the length/height of the user on the platform). The therapy system can include a second spray assembly supported by the frame and positioned vertically below the platform. The second spray system can include a plurality of spray heads configured to direct a flow of fluid in a direction towards the platform (e.g., upwards towards a user on the platform) and along a length of the platform (e.g., along the length/height of the user on the platform). The therapy system can include head and foot spray assemblies positioned at lateral ends of the platform (e.g., where a user's head and a user's feet would rest when they are on the platform) and configured to direct a flow of fluid in a direction towards the platform (e.g., towards a user on the platform). The head and foot spray assemblies can be actuated by an arm assembly to selectively adjust a direction of the flow of the fluid therefrom (e.g., to direct the flow of fluid at a particular body part or area of the body of the user on the platform).

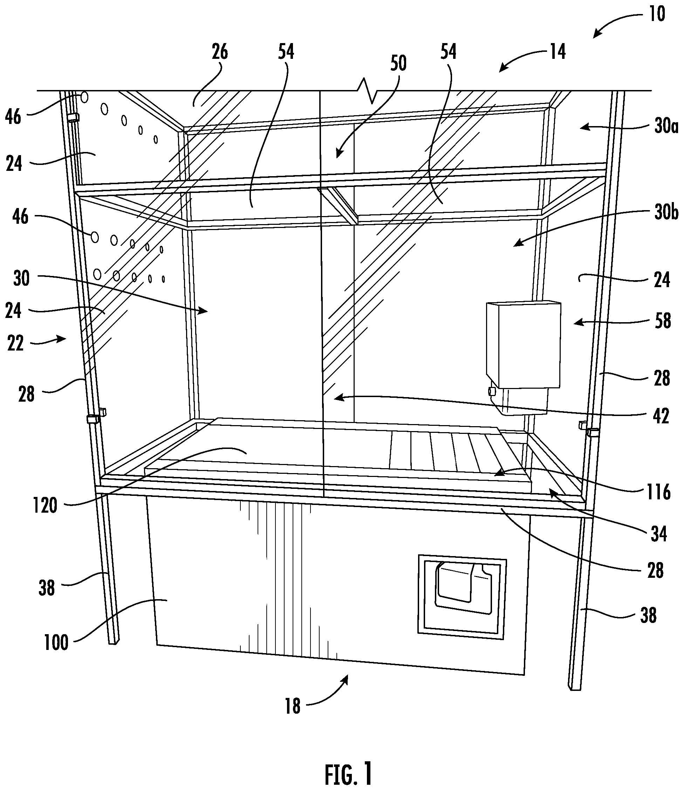

Referring to FIGS. 1-3, a therapy system (e.g., a sauna and bath assembly, a spa device, etc.) is shown as system 10, according to an embodiment. The system 10 is configured to provide a hot therapy and/or a cold therapy experience to a user (e.g., a user sitting, laying, standing inside of the system 10). The system 10 is configured to transition the experience between the hot therapy and the cold therapy. The system 10 includes a first, hot therapy portion (e.g., a heated enclosure, sauna, housing, space, etc.), shown as sauna assembly 14, and a second, cold therapy portion (e.g., bath, tub assembly, bathing environment, basin, tank, etc.), shown as bath assembly 18, positioned below the sauna assembly 14. The sauna assembly 14 is configured to provide the hot therapy experience. By way of example, the sauna assembly 14 may heat an interior chamber of the sauna assembly 14, supply steam to an interior chamber of the sauna assembly 14, supply (e.g., discharge, direct, etc.) warm water on a user within the sauna assembly 14, and/or otherwise provide the hot therapy experience. The bath assembly 18 is configured to provide the cold therapy experience. By way of example, an interior cavity (e.g., a bathing volume) of bath assembly 18 can be filled and hold a supply of water (e.g., ice water, cold water, etc.) in which the user can at least partially submerge such that the bath assembly 18 provides the cold therapy experience. In other embodiments, the supply of water held by the interior cavity of the bath assembly 18 can be warm such that the bath assembly 18 provides the hot therapy experience.

The sauna assembly 14 includes a frame 22 structured to support a plurality of sidewalls 24 (e.g., panels, panes, etc.) and a top wall 26 (e.g., a panel, a pane, etc.). The frame 22 includes a plurality of frame members 28 to support the sidewalls 24 and the top wall 26. The frame members 28 may be variously arranged (e.g., oriented) vertically and horizontally to support both the sidewalls 24 and the top wall 26. The sidewalls 24 extend in substantially vertical planes (e.g., within 5% of vertical, substantially planar, etc.) between the frame members 28 of the frame 22. The sidewalls 24 and the top wall 26 collectively define an enclosure (e.g., space, chamber, cavity, closed space, etc.), shown as interior chamber 30, of the sauna assembly 14.

The sauna assembly 14 includes an opening 34 along a bottom portion (e.g., plane, lower horizontal plane, etc.) of the interior chamber 30. The opening 34 may be sized to receive at least a portion of the bath assembly 18. The opening 34 facilitates access between the interior chamber 30 and a surrounding, exterior environment. By way of example, the sauna assembly 14 does not include a bottom wall such that the opening 34 is defined along the bottom portion of the interior chamber 30. By way of another example, the sidewalls 24 are spaced apart from each other such that the frame members 28 outline the opening 34. In some embodiments, the system 10 does not include the opening 34. In such embodiments, the interior chamber 30 may be isolated (e.g., closed, separated, etc.) from the exterior environment. By way of example, the sauna assembly 14 may be positioned along a ground surface or include a bottom wall (e.g., floor, floor surface, etc.) to isolate the interior chamber 30 from the exterior environment.

The sidewalls 24 may extend between the top wall 26 and the opening 34. The top wall 26 extends perpendicular to the sidewalls 24 in a substantially horizontal plane (e.g., within 5% of horizontal, parallel to a ground surface on which the system 10 stands) proximate upper edges of the sidewalls 24. The sidewalls 24 and the top wall 26 may form an insulated seal (e.g., with the frame members 28) between the interior chamber 30 of the sauna assembly 14 and an exterior environment surrounding the sauna assembly 14 to control (e.g., regulate) the conditions (e.g., temperature, humidity, etc.) within the interior chamber 30.

The frame 22 includes a plurality of legs 38 extending in a substantially vertical direction. The legs 38 are configured to engage with a ground surface and support the sidewalls 24 and the top wall 26 such that the interior chamber 30 is positioned a certain height above the ground surface. By way of example, the legs 38 may extend from bottom corners of the frame 22 in a direction between the ground surface and the frame members 28 proximate the opening 34. In some embodiments, the frame 22 does not include the legs 38.

The sauna assembly 14 includes a door 42 configured to move (e.g., pivot, slide, rotate, etc.) between an open position and a closed position to facilitate selective access to the interior chamber 30. By way of example, a user may move the door 42 from the closed position to the open position such that a space is provided (e.g., a space between the frame members 28, a space between one of the sidewalls 24, etc.) for the user to enter the interior chamber 30 (e.g., enter the sauna assembly 14, enter the bath assembly 18, enter the system 10, etc.) to experience the hot and/or cold therapy experience. Similarly, when the user is inside the sauna assembly 14, they may move the door 42 between the open and closed positions. The door 42 may be positioned between the frame members 28 and may be supported thereby. In some embodiments, one or more of the sidewalls 24 are configured as the door 42. By way of example, the door 42 may be positioned in place of one of the sidewalls 24. The door 42 may form an insulated seal (e.g., with one or more of the frame members 28, with the one or more of the sidewalls 24, with the top wall 26, etc.) between the interior chamber 30 of the sauna assembly 14 and an exterior environment surrounding the sauna assembly 14 to control (e.g., regulate) the conditions (e.g., temperature, humidity, etc.) within the interior chamber 30.

As shown in FIGS. 1-3, the sidewalls 24 include one or more apertures (e.g., holes, outlets/inlets, openings, flues, slits, grilles, louvers, etc.), shown as vents 46. The vents 46 may be variously positioned along the surface of one or more of the sidewalls 24. The vents 46 permit the flow of fluids (e.g., air, steam, water, etc.) between the interior chamber 30 and the exterior environment surrounding the sauna assembly 14. The vents 46 may be provided to control (e.g., regulate) the conditions (e.g., temperature, humidity, etc.) within the interior chamber 30. In some embodiments, one or more vents 46 are positioned along the surface of the top wall 26. In some embodiments, the system 10 does not include the vents 46.

As shown in FIGS. 1-3, a separator (e.g., a separation portion, a wall, a sliding window, partition, divider, etc.), shown as separator 50, is provided within the interior chamber 30 of the sauna assembly 14. The separator 50 may divide the interior chamber 30 into sub-interior chambers. By way of example, the separator 50 may divide the interior chamber 30 into a first interior chamber 30a (e.g., an upper interior chamber) and a second interior chamber 30b (e.g., a lower interior chamber). The first interior chamber 30a may be formed, collectively, by a first (e.g., upper) portion of the sidewalls 24, the top wall 26, and the separator 50 (e.g., an upward facing surface of the separator 50). The second interior chamber 30b may be formed, collectively, by a second (e.g., lower) portion of the sidewalls 24 and the separator 50 (e.g., a downward facing surface of the separator 50).

The separator 50 may extend within a horizontal plane parallel with and positioned below the top wall 26. The separator 50 may include two panels (e.g., panes, walls, surfaces, etc.), shown as panels 54, supported by the frame members 28. As shown, the panels 54 are separated by a horizontal frame member 28 extending therebetween. In some embodiments, the panels 54 are removable from the separator 50 to facilitate fluidly coupling the first interior chamber 30a and the second interior chamber 30b. In other embodiments, one or both of the panels 54 are configured to selectively move (e.g., pivot, slide, rotate, etc.) between an open position and a closed position to facilitate fluidly coupling the first interior chamber 30a and the second interior chamber 30b. Removing or otherwise moving one or both of the panels 54 increases the volume of the first interior chamber 30a such that a larger space is provided for a user to stand, sit, or move within the interior chamber 30 of the sauna assembly 14. In some embodiments, the separator 50 includes more or fewer than two panels 54. In some embodiments, the system 10 does not include the separator 50 such that the interior chamber 30 is a single, unseparated volume.

As shown in FIGS. 1, 2, 5, and 6, the sauna assembly 14 includes a sauna element 58 in fluid communication with the interior chamber 30. In some embodiments, the sauna element 58 is a steam generator configured to generate steam and provide the steam to the interior chamber 30. In other embodiments, the sauna element 58 is a heater configured to provide heated air to the interior chamber 30 or otherwise heat the air within the interior chamber 30. In yet other embodiments, the sauna element 58 is configured as or otherwise includes both a heater and a steam generator. In such embodiments, the sauna element 58 may be configured to selectively (i) generate and provide steam to the interior chamber 30 and/or (ii) provide heated air to or otherwise heat the air within the interior chamber 30.

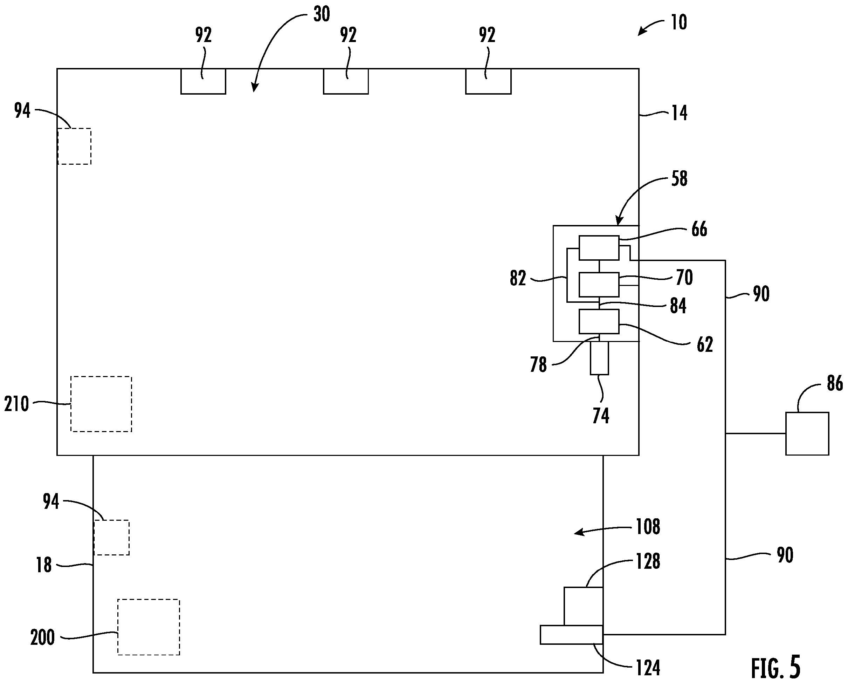

Referring to FIG. 5, the sauna element 58 includes a blower 62 (e.g., air blower, air pump, etc.) fluidly coupled to the interior chamber 30; a steam generator 66 configured to generate steam; a heating element 70 (e.g., a heat transfer member, a resistive heating coil, etc.) configured to heat a supply of air; and one or more nozzles 74 fluidly coupled with the interior chamber 30 and the blower 62. The blower 62 may be fluidly coupled with the interior chamber 30 by a first conduit 78 and fluidly coupled with the steam generator 66 by a second conduit 82. The blower 62 is configured to receive a flow of air from an air supply source, such as the interior chamber 30, although the flow of air may be received from a different air supply source, such as ambient, according to other exemplary embodiments. The blower 62 may be configured to direct the flow of air to or receive the flow of air from the steam generator 66 via the second conduit 82. The heating element 70 is configured to heat the flow of air and provide a heated flow of air via a third conduit 84 to the blower 62 and the interior chamber 30 to adjust the temperature of the interior chamber 30.

Referring to FIG. 5, the steam generator 66 is fluidly coupled to a fluid supply source 86, such as a household water supply, although other fluid supply sources may be used, according to other exemplary embodiments. The steam generator 66 is fluidly coupled to the fluid supply source 86 by a fourth conduit 90. The steam generator 66 is configured to receive a flow of water from the fluid supply source 86 and heat the received water via a heating element included therein to generate steam. In such an embodiment, the steam is delivered to the blower 62 and/or the nozzle 74 by the second conduit 82 to be discharged into the interior chamber 30. Further, in such an embodiment, the heating element 70 is configured to heat a flow of air and provide a heated flow of air to the interior chamber 30 (e.g., via a nozzle 74 that is different than the nozzle 74 used to deliver the steam). In other embodiments, the steam generator 66 supplies the flow of water to the intermediate heating element 70 to heat the water, thereby generating steam. In some embodiments, the steam generator 66 is configured to direct the generated steam to the interior chamber 30, where the steam can be combined with the heated air generated by the heating element 70. The steam may be provided to selectively increase the humidity of the interior chamber 30. In this manner, the sauna element 58 can selectively and separately provide heated air and steam to the interior chamber 30 (e.g., a sauna or shower environment) to provide control of temperature and humidity within the interior chamber 30, thereby providing the hot therapy experience. In some embodiments, the steam generator 66 (e.g., the sauna element 58) includes a diffuser configured to provide an aroma (e.g., by way of scented oils) to the interior chamber 30. The thermal element of the steam generator 66 may facilitate the release of the aroma/scent by vaporizing the essential oils. The vaporized essential oils may be supplied (e.g., diffused, supplied) to the generated steam and discharged to the interior chamber 30 via the nozzles 74. The aroma may induce a relaxing or calming sensation experienced by the user thereby promoting the experience.

According to an exemplary embodiment, the system 10 includes one or more lighting elements 92 (e.g., infrared heat lamps, halogen heat lamps, heat-producing LED bulbs, UVB lights, etc.) configured to emit light to illuminate the interior chamber 30 and convectively or radiatively heat the air/steam within the interior chamber 30.

According to an exemplary embodiment, the system 10 includes at least one senor (e.g., temperature sensor, humidity sensor, etc.), shown as sensor 94, configured to monitor a condition of the sauna assembly 14 and/or the bath assembly 18. By way of example, a temperature sensor 94 and a humidity sensor 94 may be positioned within the interior chamber 30 of the sauna assembly 14 to monitor the temperature and the humidity, respectively, of the air/steam within the interior chamber 30. By way of another example, a temperature sensor 94 may be positioned within a bathing volume (e.g., bathing volume 108) of the bath assembly 18 to monitor the temperature of the fluid held within the bathing volume.

Referring back to FIGS. 1-3, the bath assembly 18 is configured to be positioned below the sauna assembly 14. In some embodiments, the bath assembly 18 is positioned below the sauna assembly 14 such that at least a portion of the extends through the opening 34 of the sauna assembly 14 and into the interior chamber 30. The bath assembly 18 includes a base 100 (e.g., an outer shell) configured to house and support one or more components of the bath assembly 18. The bath assembly 18 includes an internal shdl 104 (e.g., tub, basin, etc.) defining a bathing volume 108 (e.g., cavity) structured to receive and hold a fluid (e.g., water, bathing solution, etc.). By way of example, the bathing volume 108 can be filled with a supply of water. The bathing volume 108 is sized to receive a user such that the user can sit, lay, or stand in the bathing volume 108 of the internal shell 104.

An upper peripheral edge of the base 100 and/or internal shell 104 can define a shoulder 112 (e.g., support structure, lip, flange, etc.) configured to support a support structure (e.g., bath, bed, seat, support surface, platform, etc.), shown as cover 116. In some embodiments, the shoulder 112 extends along the entire upper peripheral edge of the base 100 and/or internal shell 104. In other embodiments, the shoulder 112 extends along opposing (e.g., left and right, front and back, etc.) upper peripheral edges of the base 100 and/or internal shell 104. In some embodiments, the shoulder 112 is recessed within the bathing volume 108 such that the cover 116 is substantially flush with the upper peripheral edge of the internal shell 104.

In some embodiments, the cover 116 is formed as a single unitary body (e.g., as a monolithic piece that cannot be separated without damaging the cover 116). In other embodiments, the cover 116 is formed from two or more connected sheets, slats, portions, etc. In such embodiments, the cover 116 may include a plurality of slats coupled together with a flexible material or backing such that the cover 116 can roll about itself (e.g., into a roll). The cover 116 may be made from a material such as wood, plastic, metal, and/or any other suitable material or combination of materials. In some embodiments, the cover 116 includes a seat portion, shown as cushion 120. The cushion 120 may be made from a pliable (e.g., malleable) material such as rubber (or another suitable material) to support the user. By way of example, when the user is supported by the cover 116 and the cushion 120, the cushion 120 may conform to the contours of the portions of the user (e.g., body parts) supported thereby.

The cover 116 may engage with and be supported by the shoulder 112 such that the cover 116 covers (e.g., encloses) the bathing volume 108 defined by the internal shell 104. By way of example, when the bath assembly 18 is in a covered configuration (e.g., when the cover 116 is installed), access to the bathing volume 108 from the interior chamber 30 of the sauna assembly 14 is inhibited. The shoulder 112 may support the cover 116 (e.g., when the bath assembly 18 is in the covered configuration) such that a user can sit, lay, or stand on the cover 116 without entering the bathing volume 108.

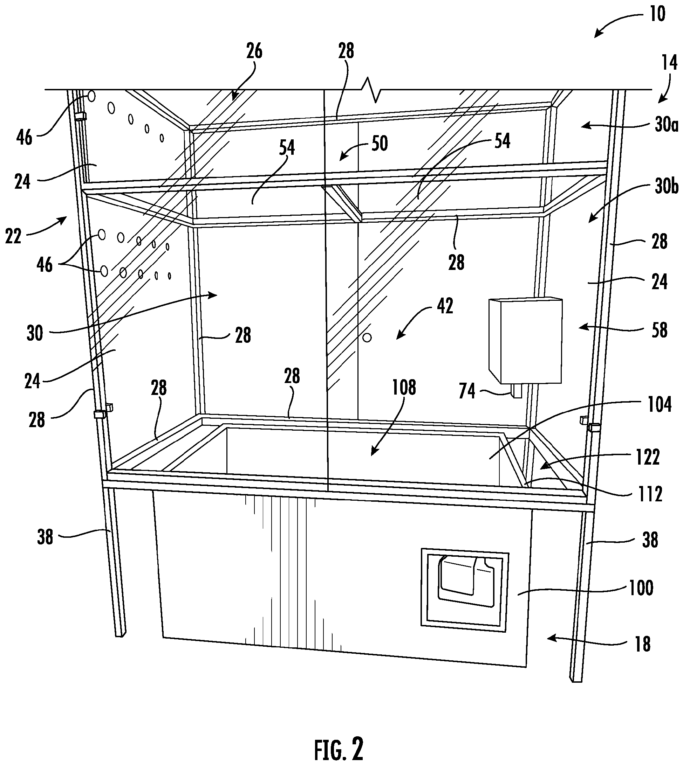

The cover 116 may be selectively removed from the shoulder 112 to transition the bath assembly 18 to an uncovered configuration. As shown in FIG. 2, the bath assembly 18 is in the uncovered configuration. In the uncovered configuration, the cover 116 is removed or otherwise repositioned such that access to the bathing volume 108 from the interior chamber 30 of the sauna assembly 14 is permitted. In such a configuration, a user can freely enter the bathing volume 108 to submerge in the fluid held within, and thereby experience the cold therapy experience (or the hot therapy experience if the fluid is warm/hot). In some embodiments, such as when the cover 116 is made from a plurality of slats coupled together with a flexible material or backing such that the cover 116 can roll about itself (e.g., into a roll), an actuation mechanism may automatically roll up or otherwise retract/extend the cover 116 to transition the bath assembly 18 between the covered and the uncovered configurations. In some embodiments, a portion (e.g., half) of the cover 116 may be selectively removed from the shoulder such that a first portion of the bathing volume 108 is covered by the cover 116 and a second portion of the bathing volume 108 is uncovered. In such embodiments, the user may sit on the portion of the cover 116 covering the bathing volume 108 such that a first portion of the user (e.g., a portion of the upper body of the user) is disposed within the interior chamber 30 of the sauna assembly 14 and a second portion of the user (e.g., a portion of the lower body of the user) is submerged in the fluid filling the bathing volume 108.

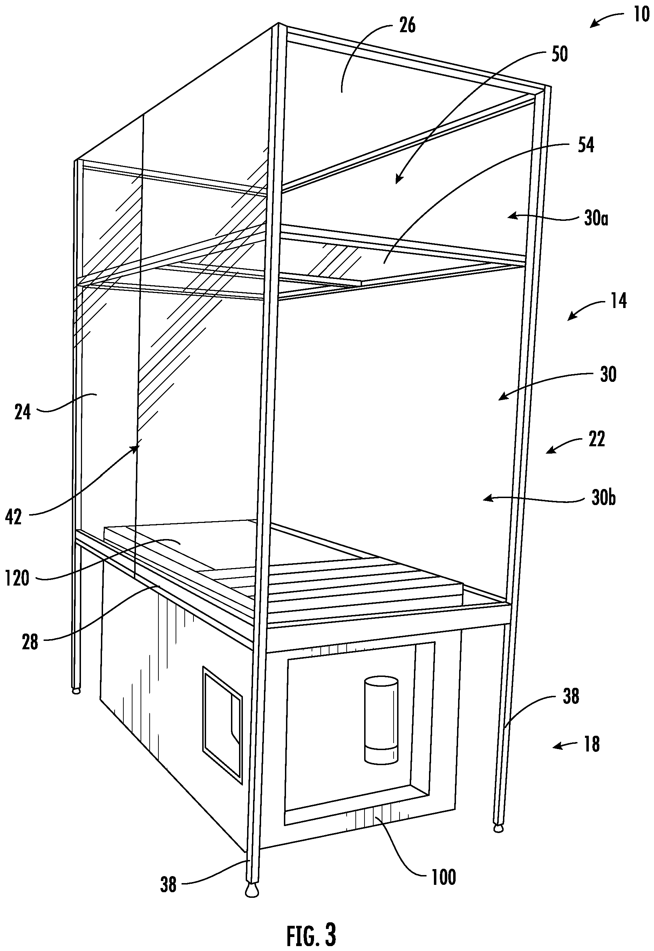

As shown in FIGS. 1 and 3, the bath assembly 18 is positioned below the sauna assembly 14 such that at least a portion of the bath assembly 18 is positioned within (e.g., received within) the interior chamber 30. As shown, when the bath assembly 18 is in the covered configuration and the cover 116 is engaged with the shoulder 112, because of the positioning of the bath assembly 18 relative to the sauna assembly 14, the user can sit, lay, or stand on the cover 116 and be within the interior chamber 30 of the sauna assembly 14. In such an embodiment, the cover 116 acts as a platform structured to support the user (e.g., the weight of the user) inside of the interior chamber 30 such that the user can experience the hot therapy experience.

As shown in FIG. 2, the bath assembly 18 may be transitioned to the uncovered position such that a user can freely enter the bathing volume 108 to submerge in the fluid held within, and thereby experience the cold therapy experience. In this manner, the system 10 facilitates providing the hot therapy experience and the cold therapy experience.

In some embodiments, an insulation member 122 is positioned between the bath assembly 18 and the sauna assembly 14 (e.g., within the opening 34 between the portion of the bath assembly 18 extending withing the interior chamber 30 and the sauna assembly 14). The insulation member 122 may be configured to form an insulated seal (e.g., with the base 100 and/or internal shell 104 of the bath assembly 18 and the frame members 28 of the sauna assembly 14) between the interior chamber 30 of the sauna assembly 14 and an exterior environment surrounding the sauna assembly 14 and the bath assembly 18 to control (e.g., regulate) the conditions (e.g., temperature, humidity, etc.) within the interior chamber 30.

As shown in FIG. 5, the bath assembly 18 is fluidly coupled with the fluid supply source 86 via the fourth conduit 90. The bath assembly 18 may include a control valve 124 fluidly coupling the bathing volume 108 with the fluid supply source 86. The control valve 124 is configured to receive a flow of fluid from the fluid supply source 86 and selectively supply the fluid to the bathing volume 108 to fill the bathing volume 108 with the fluid. The bath assembly 18 may include a thermal element 128 (e.g., arefrigerator, a cooling element) coupled with the control valve 124 and/or the fourth conduit 90 and configured to cool the fluid supplied to the bathing volume 108, thereby providing the cold therapy experience to the user submerged in the fluid. In some embodiments, the thermal element 128 is configured to heat the fluid supplied to the bathing volume 108, thereby providing the hot therapy experience to the user submerged in the fluid.

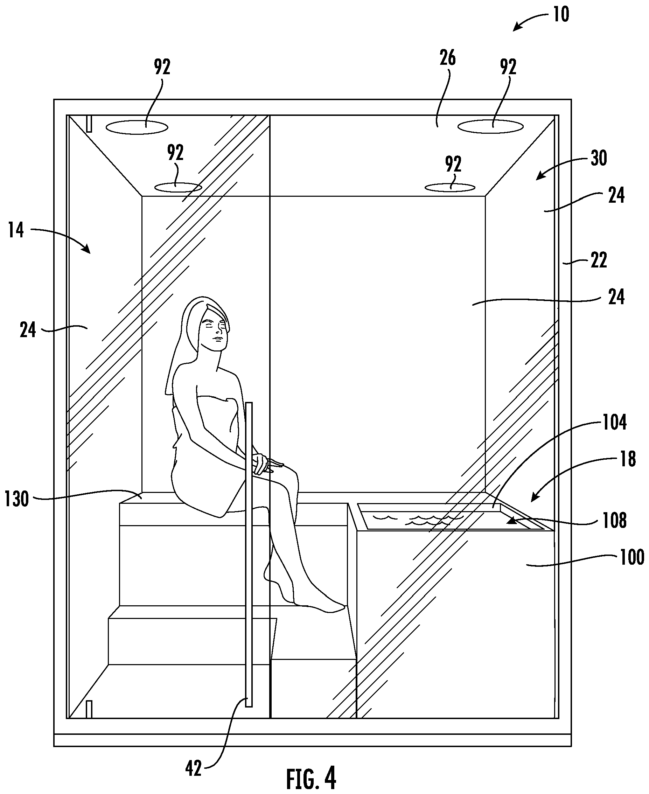

As shown in FIG. 4, the bath assembly 18 is positioned within the interior chamber 30 defined by the sidewalls 24 and the top wall 26. The system 10 shown in FIG. 4 includes a support structure 130 (e.g., seat, bench, step, etc.) positioned within the interior chamber 30 and configured to support a user within the interior chamber 30. The support structure 130 is positioned adjacent to the base 100. The user can sit, lay, or stand on the support structure 130 and be within the interior chamber 30 of the sauna assembly 14 such that the user can experience the hot therapy experience. The base 100 is positioned entirely within the interior chamber 30 such that the user may be able to selectively transition between sitting, laying, or standing on the support structure 130 to experience the hot therapy experience and entering the bathing volume 108 of the base 100 to submerge in the fluid held therein, and thereby experience the cold therapy experience. In other words, the bathing volume 108 is accessible from the interior chamber 30. In some embodiments, the user can sit on a portion of support structure 130 such that a first portion of the user (e.g., a portion of the upper body of the user) is disposed within the interior chamber 30 of the sauna assembly 14 and a second portion of the user (e.g., a portion of the lower body of the user) is submerged in the fluid filling the bathing volume 108. In some embodiments, the system 10 of FIG. 4 includes the cover 116 to selectively cover the bathing volume 108. Referring to FIG. 4, the system 10 may be substantially similar to and perform similar operations as the system 10 described above with respect to FIGS. 1-3, the entire description of which is incorporated herein. In some embodiments, the system 10 of FIG. 4 includes all the components, assemblies, and systems of the system 10 of FIGS. 1-3.

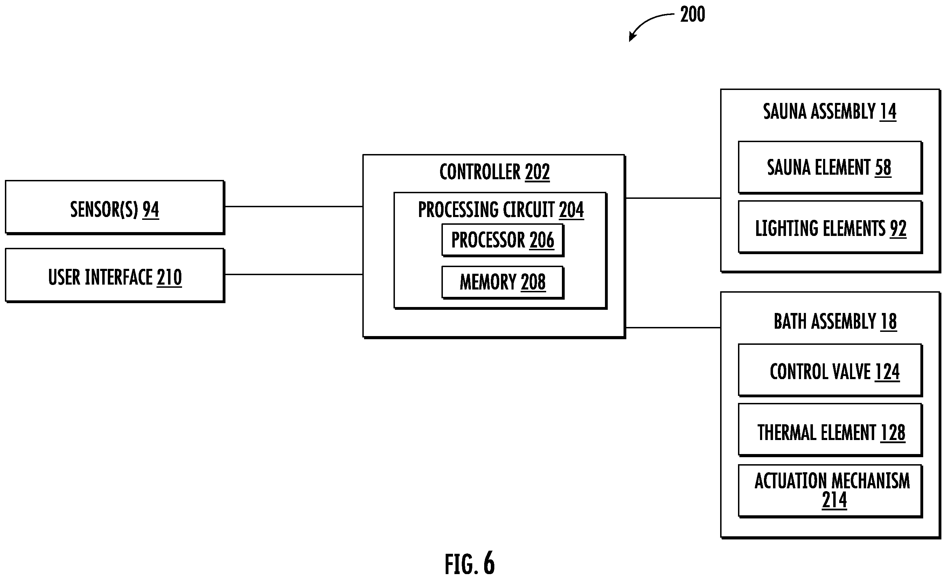

Referring to FIG. 6, the system 10 includes a control system 200 configured to control operation of the sauna assembly 14 and the bath assembly 18. The control system 200 is configured to control operation of the sauna assembly 14 and the bath assembly 18 based on user inputs and sensor signals.

The control system 200 includes a controller 202 that is communicably coupled to the sauna assembly 14 and the bath assembly 18. The controller 202 includes a processing circuit 204 having a processor 206 and memory 208. The control system 200 further includes user controls (e.g., a graphical user interface (GUI)), shown as user interface 210, communicably coupled to the controller 202. In other embodiments, the control system 200 may include additional, fewer, and/or different components.

In some embodiments, the processing circuit 204 may be a general purpose or specific purpose processor, an application specific integrated circuit (ASIC), one or more field programmable gate arrays (FPGAs), a group of processing components, or other suitable processing components. The processor 206 is configured to execute computer code or instructions stored in the memory 208 or received from other computer readable media (e.g., CDROM, network storage, a remote server, etc.).

The memory 208 may include one or more devices (e.g., memory units, memory devices, storage devices, etc.) for storing data and/or computer code for completing and/or facilitating the various processes described in the present disclosure. The memory 208 may include random access memory (RAM), read-only memory (ROM), hard drive storage, temporary storage, non-volatile memory, flash memory, optical memory, or any other suitable memory for storing software objects and/or computer instructions. The memory 208 may include database components, object code components, script components, or any other type of information structure for supporting the various activities and information structures described in the present disclosure. The memory 208 may be communicably connected to the processor 206 via the processing circuit 204 and may include computer code for executing one or more processes described herein.

The user interface 210 may be a human-machine interface that is configured to provide an indication of an operating status and/or control information regarding the system 10 to a user. The user interface 210 is also configured to receive and interpret user commands. In some embodiments, the user interface 210 is part of the controller 202, such as a software module stored in the memory 208 that may be generated by the controller 202 and transmitted to a user device (e.g., a mobile phone, a tablet, a computer, etc.). In other embodiments, the user interface 210 may be part of a software program or mobile application that can be linked to the control system 200 to control operation of the sauna assembly 14 and the bath assembly 18. In yet other embodiments, the user interface 210 is a physical device such as a mobile phone, a tablet, a computer, a knob, a dial, a thermostat, button, etc. configured to receive an input from the user and transmit a signal indicative of the input to the controller 202. In such an embodiment, the user interface 210 may be positioned within the interior chamber 30, along the base 100, or otherwise positioned such that the user can reach the user interface 210 (e.g., when the user is inside the interior chamber 30, when the user is submerged in the fluid held within the bathing volume 108, etc.).

In some embodiments, the controller 202 uses the user interface 210 to receive input from various sensors 94 and send control signals to various components of the sauna assembly 14 and/or the bath assembly 18 based on the received input. In such an embodiment, the sensors 94 are communicably coupled with the controller 202 and configured to transmit sensor signals to the controller 202 indicative of the conditions within the interior chamber 30 and/or the bathing volume 108. By way of example, the sensor 94 configured as a temperature sensor may send a signal to the controller 202 indicative of the temperature measured by the sensor 94 (e.g., a temperature of the air in the interior chamber 30, a temperature of the fluid in the bathing volume 108). In response to receiving the signal, the controller 202 may transmit a control signal to the sauna assembly 14 and/or the bath assembly 18. The control signal may control an output of the heating element 70 and/or the thermal element 128 to control a temperature of the air/steam in the interior chamber 30 and the temperature of the fluid in the bathing volume 108, respectively. By way of example, in response to receiving the sensor signal, the controller 202 may determine that the temperature is above or below a predetermined temperature threshold. Based on the determination, the controller 202 may send a control signal to control an output of the heating element 70 and/or the thermal element 128 to control a temperature of the air/steam in the interior chamber 30 and the temperature of the fluid in the bathing volume 108, respectively, to a desired temperature.

By way of another example, the sensor 94 configured as a humidity sensor may send a signal to the controller 202 indicative of the humidity measured by the sensor 94 (e.g., a humidity of the air in the interior chamber 30). In response to receiving the signal, the controller 202 may transmit a control signal to the sauna assembly 14. The control signal may control an output of the steam generator 66 to control a humidity of the air in the interior chamber 30.

In some embodiments, the user can provide an input to the user interface 210 to control operation of the sauna element 58. By way of example, in response to receiving an input signal, the controller 202 can control operation of the blower 62, the steam generator 66, the heating element 70, and the nozzles 74. In response to receiving a control signal indicative of the input signal, the flow rate from the blower 62 through the nozzles 74 can be selectively controlled to adjust the temperature/humidity of the interior chamber 30. In response to receiving a control signal indicative of the input signal, the output from the steam generator 66 can be selectively controlled to adjust the temperature/humidity of the interior chamber 30. In response to receiving a control signal indicative of the input signal, the temperature output from the heating element 70 (e.g., the resistance through the heating element 70) can be selectively controlled to adjust the temperature of the interior chamber 30.

In some embodiments, the user can provide an input to the user interface 210 to control operation of the lighting elements 92. By way of example, in response to receiving an input signal, the controller 202 can turn the lighting elements 92 ON or OFF. In some embodiments, the lighting elements 92, in response to receiving a control signal indicative of the input signal, are configured to emit one or more patterns of light including one or more colors of light.

In some embodiments, the user can provide an input to the user interface 210 to control operation of the control valve 124. By way of example, in response to receiving an input signal, the controller 202 can actuate the control valve 124 between an open position to supply fluid from the fluid supply source 86 to the bathing volume 108 and a closed position to inhibit fluid from being supplied from the fluid supply source 86 to the bathing volume 108.

In some embodiments, the user can provide an input to the user interface 210 to control operation of the thermal element 128. By way of example, in response to receiving an input signal, the controller 202 can control operation of the thermal element 128. In response to receiving a control signal indicative of the input signal, the temperature output from the thermal element 128 (e.g., the resistance through the thermal element 128, the operation of a compressor of the refrigerator) can be selectively controlled to adjust the temperature of the fluid supplied to the bathing volume 108.

In some embodiments, the user can provide an input to the user interface 210 to control operation of an actuation mechanism 214 (e.g., a motor assembly). The actuation mechanism 214 may be mechanically coupled with the cover 116 and configured to retract/extend or otherwise reposition the cover 116 to transition the bath assembly 18 between the covered and the uncovered configurations. In response to receiving a control signal indicative of the input signal, the actuation mechanism 214 (e.g., a motor thereof) can be selectively controlled to adjust the position of the cover 116 to transition the bath assembly 18 between the covered and the uncovered configurations.

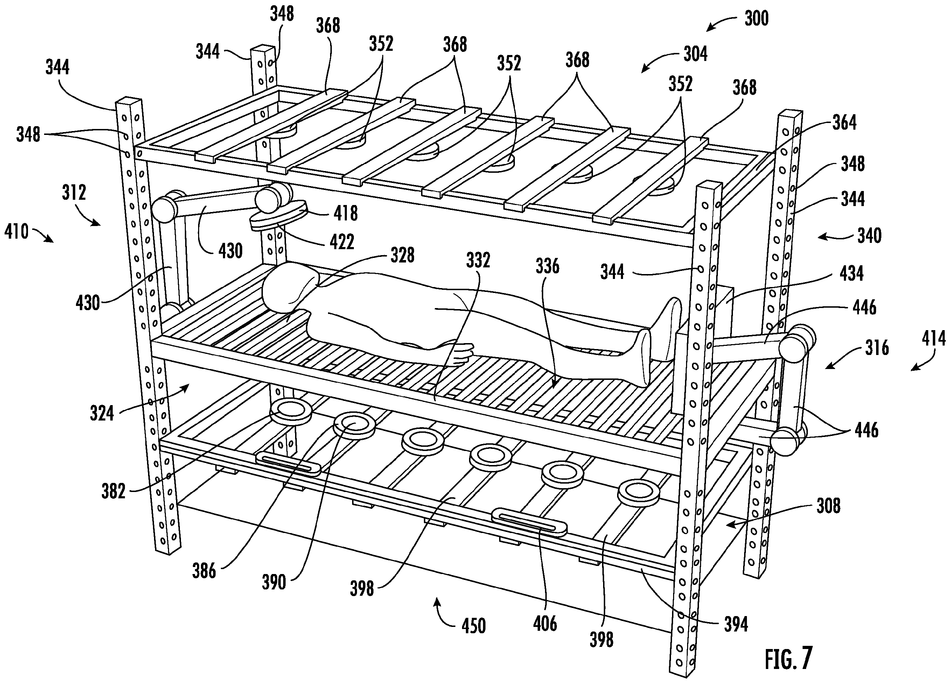

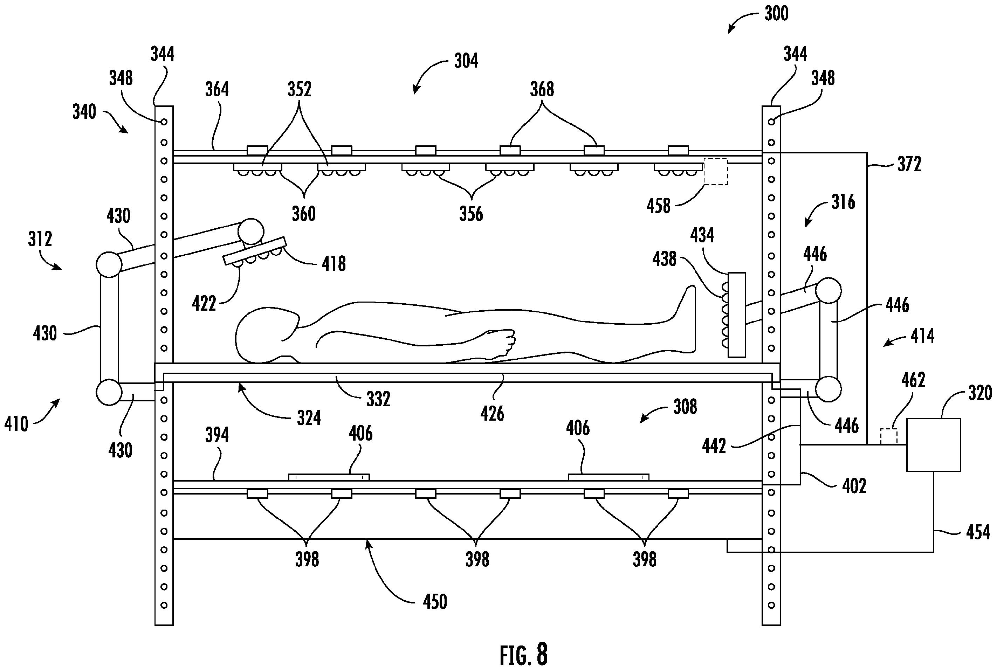

Referring to FIGS. 7 and 8, a therapy system (e.g., a shower system, a spa assembly, etc.) is shown as system 300, according to an embodiment. The system 300 is configured to selectively spray (e.g., discharge, dispense, eject, propel, etc.) a fluid (e.g., water, softened water). The system 300 may be utilized by a user (e.g., shower user, individual, etc.). By way of example, the system 300 may be utilized to spray fluid onto the user or an object (e.g., an item to be cleaned). In this manner, the usermay utilize the system 300 to cleanse the user's body (e.g., hair, body parts, etc.) and provide a therapy experience (e.g., spray hot/warm water/mist to provide a hot therapy experience, spray cold water/mist to provide a cold therapy experience).

According to an exemplary embodiment, the system 300 includes a first spray assembly (e.g., a rain panel assembly, a spray panel assembly, an overhead assembly, a first vertical spray assembly, a vichy shower assembly, etc.), shown as upper spray assembly 304; a second spray assembly (e.g., a rain panel assembly, a spray panel assembly, an underbody assembly, a second vertical spray assembly, a vichy shower assembly, etc.), shown as lower spray assembly 308; a third spray assembly (e.g., a shower head assembly, a first lateral spray assembly, a left spray assembly, etc.), shown as head spray assembly 312; and a fourth spray assembly (e.g., a shower head assembly, a second lateral spray assembly, a right spray assembly, etc.), shown as foot spray assembly 316. The upper spray assembly 304, the lower spray assembly 308, the head spray assembly 312, and the foot spray assembly 316 are fluidly coupled to and configured to receive a supply of fluid from a fluid supply source 320, such as household water supply, although other fluid supply sources may be used, according to other exemplary embodiments. The upper spray assembly 304, the lower spray assembly 308, the head spray assembly 312, and the foot spray assembly 316 are configured to discharge the fluid received from the fluid supply source 320 onto the user or an object.

The system 300 includes a support structure (e.g., a support surface, a platform, a bed, a seat, etc.), shown as platform 324, configured to support the user (e.g., the weight of the user) utilizing the system 300. The platform 324 is configured to permit the flow (e.g., spray, mist, etc.) of fluid (e.g., the fluid discharged from the upper spray assembly 304, the lower spray assembly 308, the head spray assembly 312, and the foot spray assembly 316) through the platform 324. In other words, the platform 324 is configured to allow fluid to pass therethrough. In such embodiments, when the user is disposed on (and supported by) the platform 324, the user is not submerged in the fluid because the platform 324 does not accumulate (e.g., pool, hold, store, etc.) the fluid.

The platform 324 may be made from a material and/or a plurality of components structured to permit the flow of fluid therethrough. In the embodiment shown in FIGS. 6 and 7, the platform 324 includes a plurality of slats 328 (e.g., planks, cross members, supports, etc.) extending (e.g., longitudinally extending, laterally extending, etc.) between a platform frame 332 of the platform 324. The slats 328 are shown laterally spaced apart from each other such that a plurality of gaps 336 (e.g., spaces, apertures, holes, etc.) are defined by the platform 324. In some embodiments, the slats 328 are laterally spaced apart to form the gaps 336. The slats may be made from wood (e.g., water-resistant wood), plastic, rubber, or other suitable material. The gaps 336 may be sufficiently sized to permit the flow of fluid therethrough. By way of example, the fluid can flow through the gaps 336 in a direction towards a drain assembly. By way of another example, the gaps 336 permit fluid sprayed from the lower spray assembly 308 to contact (e.g., spray, mist, massage, etc.) the user disposed on an upward facing surface of and supported by the platform 324.

In other embodiments, the slats 328 are arranged to form a pattern (e.g., a crosshatch pattern, etc.) defining the gaps 336. In yet other embodiments, the platform 324 is integrally formed as a single unitary body defining the gaps 336. In yet other embodiments, the platform 324 is a cloth, mesh, or net structure made from a material such as polyester, vinyl, cotton, nylon, or other suitable material. In such embodiments, the platform 324 may be sufficiently tensioned and supported by a frame (e.g., frame 340) of the system 300 to flex and conform to the contour of the portions of the user (e.g., body parts) supported thereby. Further, in such embodiments, the cloth, mesh, or net structure of the platform 324 permits flow of the fluid therethrough to limit pooling of the fluid.

The system 300 includes a frame 340 structured to support and couple to the upper spray assembly 304, the lower spray assembly 308, the head spray assembly 312, the foot spray assembly 316, and the platform 324. The frame 340 is configured to engage with a ground surface to space the upper spray assembly 304, the lower spray assembly 308, the head spray assembly 312, the foot spray assembly 316, and the platform 324 vertically above the ground surface. The frame 340 includes a plurality of frame members 344 vertically extending between the upper spray assembly 304 and the lower spray assembly 308. The frame members 344 are configured to engage with the ground surface on which the system 300 is disposed. The frame members 344 may include a plurality of connection points 348 (e.g., apertures, slots, notches, shoulders, etc.) along the length thereof. The connection points 348 may be configured to facilitate coupling the upper spray assembly 304, the lower spray assembly 308, and the platform 324 to the frame 340 to be supported thereby. In some embodiments, the plurality of connection points 348 along the length of the frame members 344 facilitate vertically adjusting the position of the upper spray assembly 304, the lower spray assembly 308, and the platform 324. The platform 324 may be coupled to the frame 340 such that the platform 324 extends within a substantially horizontal plane (e.g., within 5% of horizontal, parallel to a ground surface on which the system 300 stands).

Referring to FIGS. 7 and 8, the upper spray assembly 304 includes a plurality of first spray heads (e.g., shower heads, misters, overhead spray heads, etc.), shown as upper spray heads 352. The upper spray heads 352 are configured to receive a supply of fluid and discharge the fluid in a direction towards the platform 324 (e.g., the user supported by the platform 324). By way of example, the upper spray heads 352 are positioned such that they discharge the fluid downwards (e.g., vertically down) in a direction towards the platform 324 (on which the user can rest) and the ground surface. The upper spray heads 352 each include a plurality of nozzles 356 variously positioned about a spray face 360 of the upper spray heads 352 and configured to direct the fluid into a spray pattern (e.g., a spray pattern based on the arrangement of the nozzles 356), thereby discharging the fluid from the upper spray assembly 304. In some embodiments, the nozzles 356 are arranged in an annular pattern (e.g., concentric about a center point of the spray face 360). In other embodiments, the nozzles 356 are arranged in a prismed pattern about the spray face 360. In yet other embodiments, the nozzles 356 may be arranged in a different pattern about the spray face 360. In some embodiments, the flow rate of the fluid discharged through the nozzles 356 may be configurable (e.g., adjustable) to change the effect of the fluid being discharged. By way of example, the diameter of the nozzles 356 may be configurable to output a desired spray mode, such as a rainfall mode (e.g., fluid dispersed over a large area), a message mode (e.g., fluid directed at a particular area (e.g., muscle, joint) of the user), an aeration mode (e.g., a mixture of air and fluid), a mist mode (e.g., fine mist of fluid droplets), a jet mode, and/or any other modes or combination of modes. Changing the flow rate of the fluid thereby changes the pressure of the fluid discharged from the nozzles 356. In some embodiments, the upper spray heads 352 and the spray faces 360 thereof are actuatable relative to the platform 324 to change the orientation of the spray faces 360, thereby changing the direction in which the nozzles 356 discharge the fluid.

The upper spray assembly 304 includes a frame 364 configured to support the upper spray heads 352. The frame 364 is configured to couple to the frame members 344 to facilitate coupling the upper spray assembly 304 to the frame 340. The upper spray assembly 304 further includes a plurality of upper support members 368 configured to extend between and be supported by the frame 364 of the upper spray assembly 304. The upper support members 368 are configured to couple with the upper spray heads 352 to substantially center (e.g., within 5% of centered, longitudinally center, etc.) the upper spray heads 352 above the user and the platform 324. In some embodiments, each of the upper spray heads 352 are configured to couple to a single upper support member 368 extending in a lateral direction between the frame 364. The upper spray heads 352 are configured to supply fluid along a length of the platform 324 (e.g., along the length of the user, along the height of the user, from the user's head to the user's feet)

As shown in FIG. 8, the upper spray assembly 304 is fluidly coupled with the fluid supply source 320 via an upper supply conduit 372. Each of the upper spray heads 352 are fluidly coupled with each other and with the upper supply conduit 372 to receive fluid from the fluid supply source 320. In some embodiments, the upper support member 368 is hollow and defines an interior cavity to receive the upper supply conduit 372, thereby fluidly coupling each of the upper spray heads 352 with the fluid supply source 320. Similarly, in some embodiments, the frame 364 is hollow and defines an interior cavity to receive the upper supply conduit 372, thereby fluidly coupling each of the upper spray heads 352 with the fluid supply source 320. In other embodiments, the upper supply conduit 372 extends along the frame 364 and/or the upper support member 368 to fluidly couple each of the upper spray heads 352 with the fluid supply source 320.

Referring to FIGS. 7 and 8, the lower spray assembly 308 includes a plurality of second spray heads (e.g., shower heads, misters, underbody spray heads, etc.), shown as lower spray heads 382. The lower spray heads 382 are configured to receive a supply of fluid and discharge the fluid in a direction towards the platform 324 (e.g., the user supported by the platform 324). By way of example, the lower spray heads 382 are positioned such that they discharge the fluid upwards (e.g., vertically upwards) in a direction towards the platform 324 (on which the user can rest) and through the platform 324 to spray the user (e.g., through the gaps 336 of the platform 324). The lower spray heads 382 each include a plurality of nozzles 386 variously positioned about a spray face 390 of the lower spray heads 382 and configured to direct the fluid into a spray pattern (e.g., a spray pattern based on the arrangement of the nozzles 386), thereby discharging the fluid from the lower spray assembly 308. In some embodiments, the nozzles 386 are arranged in an annular pattern (e.g., concentric about a center point of the spray face 390). In other embodiments, the nozzles 386 are arranged in a prismed pattern about the spray face 390. In yet other embodiments, the nozzles 386 may be arranged in a different pattern about the spray face 390. In some embodiments, the flow rate of the fluid discharged through the nozzles 386 may be configurable (e.g., adjustable) to change the effect of the fluid being discharged. By way of example, the diameter of the nozzles 386 may be configurable to output a desired spray mode, such as a rainfall mode (e.g., fluid dispersed over a large area), a message mode (e.g., fluid directed at a particular area (e.g., muscle, joint) of the user), an aeration mode (e.g., a mixture of air and fluid), a mist mode (e.g., fine mist of fluid droplets), a jet mode, and/or any other modes or combination of modes. Changing the flow rate of the fluid thereby changes the pressure of the fluid discharged from the nozzles 386. In some embodiments, the lower spray heads 382 and the spray faces 390 thereof are actuatable relative to the platform 324 to change the orientation of the spray faces 390, thereby changing the direction in which the nozzles 386 discharge the fluid.

The lower spray assembly 308 includes a frame 394 configured to support the lower spray heads 382. The frame 394 is configured to couple to the frame members 344 to facilitate coupling the lower spray assembly 308 to the frame 340. The lower spray assembly 308 further includes a plurality of lower support members 398 configured to extend between and be supported by the frame 394 of the lower spray assembly 308. The lower support members 398 are configured to couple with the lower spray heads 382 to substantially center (e.g., within 5% of centered, longitudinally center, etc.) the lower spray heads 382 below the user and the platform 324. In some embodiments, each of the lower spray heads 382 are configured to couple to a single lower support member 398 extending in a lateral direction between the frame 394. The lower spray heads 382 are configured to supply fluid along a length of the platform 324 (e.g., along the length of the user, along the height of the user, from the user's head to the user's feet).

As shown in FIG. 8, the lower spray assembly 308 is fluidly coupled with the fluid supply source 320 via a lower supply conduit 402. Each of the lower spray heads 382 are fluidly coupled with each other and with the lower supply conduit 402 to receive fluid from the fluid supply source 320. In some embodiments, the lower support member 398 is hollow and defines an interior cavity to receive the lower supply conduit 402, thereby fluidly coupling each of the lower spray heads 382 with the fluid supply source 320. Similarly, in some embodiments, the frame 394 is hollow and defines an interior cavity to receive the lower supply conduit 402, thereby fluidly coupling each of the lower spray heads 382 with the fluid supply source 320. In other embodiments, the lower supply conduit 402 extends along the frame 394 and/or the lower support member 398 to fluidly couple each of the lower spray heads 382 with the fluid supply source 320.

The lower spray assembly 308 may further include at least one spray head 406 positioned along the frame 394 and fluidly coupled with the fluid supply source 320. The spray head 406 may include a slit nozzle configured to direct the received fluid in a direction towards the user. In some embodiments, the lower spray assembly 308 does not include the at least one spray head 406.

As shown in FIGS. 7 and 8, the head spray assembly 312 is coupled to the platform frame 332 at a first end 410 (e.g., a first lateral end) thereof, and the foot spray assembly 316 is coupled to the platform frame 332 at a second end 414 (e.g., a second lateral end) thereof, the second end 414 opposite the first end 410.

The head spray assembly 312 includes a spray face 418 including a plurality of nozzles 422 extending therethrough. The nozzles 422 may be configured to perform the same or substantially similar operations as the nozzles 356 and the nozzles 386. The head spray assembly 312 is fluidly coupled to the fluid supply source 320 via a supply conduit 426. The nozzles 422 may receive a supply of fluid from the supply conduit 426 and discharge the fluid in a direction towards the platform 324 (e.g., the user supported by the platform 324).

The head spray assembly 312 and the spray face 418 thereof are actuatable relative to the platform 324 to change the orientation of the spray face 418, thereby changing the direction in which the nozzles 422 discharge the fluid. The head spray assembly 312 includes a plurality of arms 430 (e.g., arm members, arm assembly, etc.) coupled to each other and movable relative to each other. One or more of the arms 430 may be coupled to the platform frame 332 to facilitate coupling the head spray assembly 312 with the platform 324. In other embodiments, the arms 430 couple the head spray assembly 312 to the frame 340 of the system 300. The arms 430 are configured to facilitate fluidly coupling the spray face 418 to the supply conduit 426. By way of example, the arms 430 may be hollow and define an interior cavity to receive the supply conduit 426, thereby fluidly coupling the nozzles 422 with the fluid supply source 320. By way of another example, the supply conduit 426 may extend along the arms 430 to fluidly couple the nozzles 422 with the fluid supply source 320.

The arms 430 may be actuatable relative to each other to change the orientation of the spray face 418, thereby changing the direction in which the nozzles 422 discharge the fluid. By way of example, the arms 430 are pivotal relative to each other to adjust the position of the arms 430 relative to each other to maintain the arms 430 in any moved position (e.g., a moved position by the user, a moved position by an actuation mechanism, etc.). In some embodiments, the spray face 418 is coupled to the arms 430 in a telescoping manner that is adjustable to maintain the spray face 418 in any moved position. By way of example, a user my adjust the vertical, lateral, and/or longitudinal position of the arms 430 and the spray face 418 to target the fluid discharged from the nozzles 422 at a certain area (e.g., a certain body part).

The foot spray assembly 316 includes a spray face 434 including a plurality of nozzles 438 extending therethrough. The nozzles 438 may be configured to perform the same or substantially similar operations as the nozzles 356 and the nozzles 386. The foot spray assembly 316 is fluidly coupled to the fluid supply source 320 via a supply conduit 442. The nozzles 438 may receive a supply of fluid from the supply conduit 442 and discharge the fluid in a direction towards the user.

The foot spray assembly 316 and the spray face 434 thereof are actuatable relative to the platform 324 to change the orientation of the spray face 434, thereby changing the direction in which the nozzles 438 discharge the fluid. The foot spray assembly 316 includes a plurality of arms 446 (e.g., arm members arm assembly, etc.) coupled to each other and movable relative to each other. One or more of the arms 446 may be coupled to the platform frame 332 to facilitate coupling the foot spray assembly 316 with the platform 324. In other embodiments, the arms 446 couple the foot spray assembly 316 to the frame 340 of the system 300. The arms 446 are configured to facilitate fluidly coupling the spray face 434 to the supply conduit 442. By way of example, the arms 446 may be hollow and define an interior cavity to receive the supply conduit 442, thereby fluidly coupling the nozzles 438 with the fluid supply source 320. By way of another example, the supply conduit 442 may extend along the arms 446 to fluidly couple the nozzles 438 with the fluid supply source 320.

The arms 446 may be actuatable relative to each other to change the orientation of the spray face 434, thereby changing the direction in which the nozzles 438 discharge the fluid. By way of example, the arms 446 are pivotal relative to each other to adjust the position of the arms 446 relative to each other to maintain the arms 446 in any moved position (e.g., a moved position by the user, a moved position by an actuation mechanism, etc.). In some embodiments, the spray face 434 is coupled to the arms 446 in a telescoping manner that is adjustable to maintain the spray face 434 in any moved position. By way of example, a user my adjust the vertical, lateral, and/or longitudinal position of the arms 446 and the spray face 434 to target the fluid discharged from the nozzles 438 at a certain area (e.g., a certain body part).

As shown in FIG. 8, the system 300 may include a drain assembly 450 configured to collect the fluid discharged from the upper spray assembly 304, the lower spray assembly, the head spray assembly 312, and the foot spray assembly 316 and supply the discharged fluid to the fluid supply source 320 via a return conduit 454. The discharged fluid may be filtered by the fluid supply source 320 to clean the fluid (e.g., remove particulates, contaminants, chemicals, etc.) such that the fluid can be recycled and resupplied to the upper spray assembly 304, the lower spray assembly, the head spray assembly 312, and the foot spray assembly 316. In other embodiments, the drain assembly 450 includes the filter to filter the discharged fluid such that the fluid is cleaned before being received by the fluid supply source 320.

According to an exemplary embodiment, the system 300 includes one or more lighting elements 458 (e.g., infrared heat lamps, halogen heat lamps, heat-producing LED bulbs, UVB lights, etc.) configured to emit light to illuminate the system 300 and convectively or radiatively heat the air, mist, steam, etc. within the system 300.

According to an exemplary embodiment, the system 300 includes at least one senor (e.g., temperature sensor, humidity sensor, etc.), shown as sensor 462, configured to monitor the temperature of the fluid supplied to the upper spray assembly 304, the lower spray assembly, the head spray assembly 312, and the foot spray assembly 316.

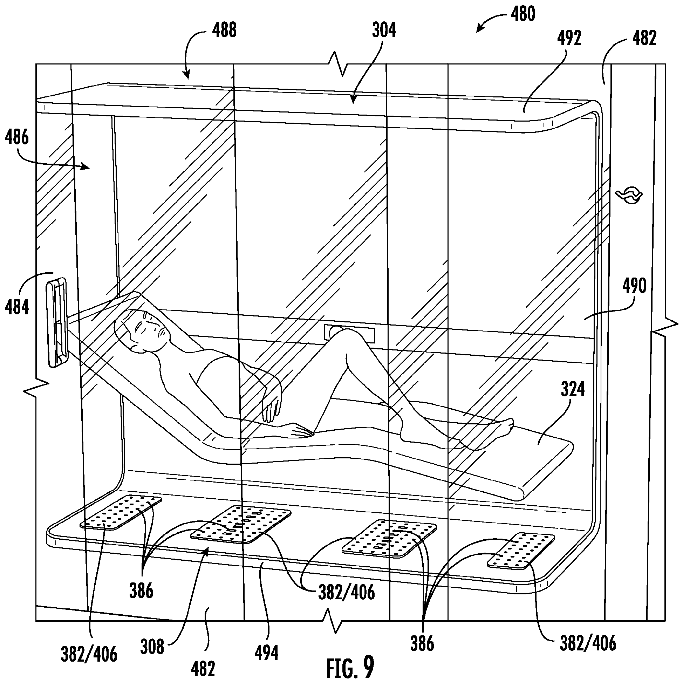

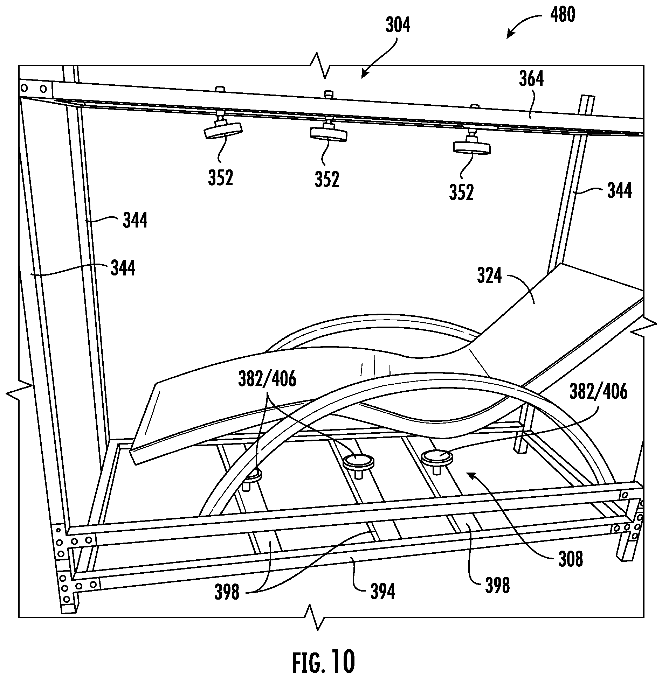

Referring to FIGS. 9 and 10, a therapy system (e.g., a shower system, a spa assembly, etc.) is shown as system 480, according to an embodiment. The system 480 may be substantially similar to and perform similar operations as the system 300 described above with respect to FIGS. 7 and 8, the entire description of which is incorporated herein. In some embodiments, the system 480 includes all the components, assemblies, and systems of the system 300 except for the head spray assembly 312 and the foot spray assembly 316 and their respective components. In other embodiments, the system 480 includes the head spray assembly 312 and the foot spray assembly 316 and their respective components. As shown in FIG. 9, the system 480 includes a plurality of walls (e.g., supports, barriers, etc.), shown as sidewalls 482, and a door 484 configured to define a chamber (e.g., interior volume, etc.), shown as interior chamber 486. The door 484 is configured to move (e.g., slide, pivot, etc.) to provide selective access to the interior chamber 486 from the exterior environment (e.g., the environment surrounding the system 480). Collectively, the sidewalls 482 and the door 484 are configured to isolate (e.g., thermally isolate, humidly isolate, etc.) the interior chamber 486 from the exterior environment (e.g., to facilitate regulating a temperature, humidity, etc., within the interior chamber 486). As shown in FIG. 10, in some embodiments, the system 480 does not include the sidewalls 482 and the door 484.

As shown in FIG. 9, the system 480 includes a support structure (e.g., support member, frame 340, etc.), shown as frame 488, positioned within the interior chamber 486. The frame 488 includes a first wall, shown as vertical wall 490, a second wall, shown as top wall 492, and a third wall, shown as bottom wall 494. The vertical wall 490 extends in a substantially vertical direction between the top wall 492 and the bottom wall 494. The vertical wall 490 is configured to support the platform 324 within the interior chamber 486. The top wall 492 extends from the vertical wall 490 in a direction vertically above the platform 324. The top wall 492 is configured to couple the upper spray assembly 304 therewith to facilitate directing fluid from the upper spray heads 352 in a direction towards the platform 324. The bottom wall 494 extends from the vertical wall 490 in a direction vertically below the platform 324. The bottom wall 494 is configured to couple the lower spray assembly 308 therewith to facilitate directing fluid from the lower spray heads 382 and/or the at least one spray head 406 in a direction towards the platform 324. In some embodiments, the vertical wall 490 forms a part of or is used in place of a sidewall of the plurality of sidewalls 482 to isolate the interior chamber 486 from the exterior environment.

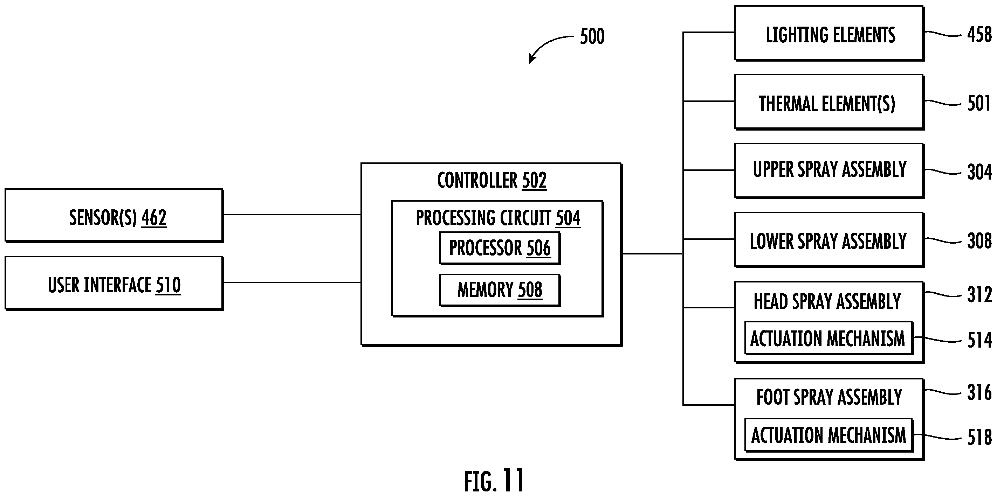

Referring to FIG. 11, a control system 500 is shown configured to control operation of the system 300 and/or the system 480. The control system 500 may be configured to control operation of the lighting elements 458, one or more thermal elements 501, the upper spray assembly 304, the lower spray assembly, the head spray assembly 312, and the foot spray assembly 316. The control system 500 is configured to control operation of various components of the system 300 and/or the system 480 based on user inputs and sensor signals.

The control system 500 includes a controller 502 that is communicably coupled to the lighting elements 458, the thermal elements 501, the upper spray assembly 304, the lower spray assembly, the head spray assembly 312, and the foot spray assembly 316. The controller 502 includes a processing circuit 504 having a processor 506 and memory 508. The control system 500 further includes a graphical user interface (GUI), shown as user interface 510, communicably coupled to the controller 502. In other embodiments, the control system 500 may include additional, fewer, and/or different components.

In some embodiments, the processing circuit 504 may be a general purpose or specific purpose processor, an application specific integrated circuit (ASIC), one or more field programmable gate arrays (FPGAs), a group of processing components, or other suitable processing components. The processor 506 is configured to execute computer code or instructions stored in the memory 508 or received from other computer readable media (e.g., CDROM, network storage, a remote server, etc.).

The memory 508 may include one or more devices (e.g., memory units, memory devices, storage devices, etc.) for storing data and/or computer code for completing and/or facilitating the various processes described in the present disclosure. The memory 508 may include random access memory (RAM), read-only memory (ROM), hard drive storage, temporary storage, non-volatile memory, flash memory, optical memory, or any other suitable memory for storing software objects and/or computer instructions. The memory 508 may include database components, object code components, script components, or any other type of information structure for supporting the various activities and information structures described in the present disclosure. The memory 508 may be communicably connected to the processor 506 via the processing circuit 504 and may include computer code for executing one or more processes described herein.

The user interface 510 may be a human-machine interface that is configured to provide an indication of an operating status and/or control information regarding the system 300 and/or the system 480 to a user. The user interface 510 is also configured to receive and interpret user commands. In some embodiments, the user interface 510 is part of the controller 502, such as a software module stored in the memory 508 that may be generated by the controller 502 and transmitted to a user device (e.g., a mobile phone, a tablet, a computer, etc.). In other embodiments, the user interface 510 may be part of a software program or mobile application that can be linked to the control system 500 to control operation of the lighting elements 458, the thermal elements 501, the upper spray assembly 304, the lower spray assembly, the head spray assembly 312, and the foot spray assembly 316. In yet other embodiments, the user interface 510 is a physical device such as a mobile phone, a tablet, a computer, a knob, a dial, a thermostat, button, etc. configured to receive an input from the user and transmit a signal indicative of the input to the controller 502. In such an embodiment, the user interface 510 may be positioned proximate the platform 324 (e.g., within the interior chamber 486), or otherwise positioned such that the user can reach the user interface 510 (e.g., when the user is supported by the platform 324, etc.).

In some embodiments, the controller 502 uses the user interface 510 to receive input from various sensors 462 and send control signals to various components of the lighting elements 458, the thermal elements 501, the upper spray assembly 304, the lower spray assembly, the head spray assembly 312, and the foot spray assembly 316 based on the received input. In such an embodiment, the sensors 462 are communicably coupled with the controller 502 and configured to transmit sensor signals to the controller 502 indicative of a temperature measured by the sensor 462 (e.g., a temperature of the fluid supplied to the upper spray assembly 304, the lower spray assembly, the head spray assembly 312, and the foot spray assembly 316). In response to receiving the signal, the controller 502 may transmit a control signal to the thermal elements 501 to control an output thereof to control a temperature of the fluid supplied to the upper spray assembly 304, the lower spray assembly, the head spray assembly 312, and the foot spray assembly 316. By way of example, in response to receiving the sensor signal, the controller 502 may determine that the temperature is above or below a predetermined temperature threshold. Based on the determination, the controller 502 may send a control signal to control an output of the thermal element 501 to control temperature of the fluid supplied to the upper spray assembly 304, the lower spray assembly, the head spray assembly 312, and/or the foot spray assembly 316 to a desired temperature.

In some embodiments, the user can provide an input to the user interface 510 to control operation of any one or combination of the upper spray assembly 304, the lower spray assembly, the head spray assembly 312, and/or the foot spray assembly 316. By way of example, in response to receiving an input signal, the controller 502 can control operation of a control valve of the upper spray assembly 304, the lower spray assembly, the head spray assembly 312, and/or the foot spray assembly 316. In response to receiving a control signal indicative of the input signal, the control valve of the upper spray assembly 304, the lower spray assembly, the head spray assembly 312, and/or the foot spray assembly 316 can be selectively controlled to selectively discharge fluid. In response to receiving a control signal indicative of the input signal, the temperature output from the thermal element 501 (e.g., the resistance through the thermal element 501) can be selectively controlled to adjust the temperature of the fluid supplied to and discharged from the upper spray assembly 304, the lower spray assembly, the head spray assembly 312, and/or the foot spray assembly 316.

In some embodiments, the user can provide an input to the user interface 510 to control operation of the lighting elements 458. By way of example, in response to receiving an input signal, the controller 502 can turn the lighting elements 458 ON or OFF. In some embodiments, the lighting elements 458, in response to receiving a control signal indicative of the input signal, are configured to emit one or more patterns of light including one or more colors of light.

In some embodiments, the user can provide an input to the user interface 210 to control operation of an actuation mechanism 514 (e.g., actuator assembly, motor assembly, etc.) of the head spray assembly 312 and/or an actuation mechanism 518 (e.g., actuator assembly, motor assembly, etc.). The actuation mechanisms 514, 518 may be mechanically coupled with the arms 430, 446 respectively, and configured to actuate or otherwise reposition the arms 430, 446 to change the orientation of the spray faces 418, 434, thereby changing the direction in which the nozzles 422, 438 discharge the fluid. In some embodiments, the actuation mechanisms 514, 518 actuate the spray faces 418, 434 in a telescoping manner to maintain the spray faces 418, 434 in any moved position. In response to receiving a control signal indicative of the input signal, the actuation mechanisms 514, 518 (e.g., motors thereof) can be selectively controlled to adjust the vertical, lateral, and/or longitudinal position of the arms 430, 446 and the spray faces 418, 434 to target the fluid discharged from the nozzles 422, 438 at a certain area (e.g., a certain body part). In some embodiments, the upper spray assembly 304 and the lower spray assembly 308 include similar actuation mechanisms to control the output and orientation of the nozzles 356 and nozzles 386, respectively. In some embodiments, the user may select a particular body part or portion of their body via the user interface 510, and in response to receiving a control signal indicative of the input signal, the actuation mechanisms 514, 518 actuate the arms 430, 446 and the spray faces 418, 434 to target the fluid discharged from the nozzles 422, 438 at the selected body part or portion of the body of the user.

As utilized herein, the terms "approximately," "about," "substantially," and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the application as recited in the appended claims.

It should be noted that the term "exemplary" as used herein to describe various embodiments is intended to indicate that such embodiments are possible examples, representations, and/or illustrations of possible embodiments (and such term is not intended to connote that such embodiments are necessarily extraordinary or superlative examples).

The terms "coupled," "connected," and the like, as used herein, mean the joining of two members directly or indirectly to one another. Such joining may be stationary (e.g., permanent) or moveable (e.g., removable or releasable). Such joining may be achieved with the two members or the two members and any additional intermediate members being integrally formed as a single unitary body with one another or with the two members or the two members and any additional intermediate members being attached to one another.

References herein to the positions of elements (e.g., "top," "bottom," "above," "below," etc.) are merely used to describe the orientation of various elements in the figures. It should be noted that the orientation of various elements may differ according to other exemplary embodiments, and that such variations are intended to be encompassed by the present disclosure.

It is important to note that the construction and arrangement of the apparatus and control system as shown in the various exemplary embodiments is illustrative only. Although only a few embodiments have been described in detail in this disclosure, those skilled in the art who review this disclosure will readily appreciate that many modifications are possible (e.g., variations in sizes, dimensions, structures, shapes and proportions of the various elements, values of parameters, mounting arrangements, use of materials, colors, orientations, etc.) without materially departing from the novel teachings and advantages of the subject matter described herein. For example, elements shown as integrally formed may be constructed of multiple parts or elements, the position of elements may be reversed or otherwise varied, and the nature or number of discrete elements or positions may be altered or varied The order or sequence of any process or method steps may be varied or re-sequenced according to alternative embodiments.

Other substitutions, modifications, changes and omissions may also be made in the design, operating conditions and arrangement of the various exemplary embodiments without departing from the scope of the present application For example, any element disclosed in one embodiment may be incorporated or utilized with any other embodiment disclosed herein.

## Claims

1. A therapy system comprising:
a first therapy assembly configured to provide a hot therapy experience, the first therapy assembly including:
a plurality of walls defining an interior chamber;
a door configured to permit selective access to the interior chamber; and
a sauna element fluidly coupled with the interior chamber and configured to provide at least one of heated air or steam to the interior chamber; and
a second therapy assembly configured to provide a cold therapy experience, the second therapy assembly including a bathtub defining a bathing volume and structured to receive and hold a fluid,
wherein the bathtub is positioned relative to the interior chamber of the first therapy assembly such that the bathing volume is accessible from the interior chamber of the first therapy assembly.

2. The therapy system of Claim 1, further comprising a support structure configured to provide selective access to the bathing volume and support a user within the interior chamber.

3. The therapy system of Claim 2, wherein the support structure is configured to transition between a covered configuration and an uncovered configuration, wherein, in the covered configuration, access to the bathing volume is inhibited, and, wherein, in the uncovered configuration, access to the bathing volume is permitted.

4. The therapy system of Claim 3, wherein, in the covered configuration, the support structure is configured to be disposed along and supported by an upper peripheral edge of the bathtub and/or, wherein transitioning the support structure between the covered configuration and the uncovered configuration facilitates transitioning the therapy system between the hot therapy experience and the cold therapy experience.

5. The therapy system of any one of the preceding Claims, wherein the bathtub is positioned at least partially within the interior chamber of the first therapy assembly.

6. The therapy system of Claim 5, wherein the second therapy assembly is positioned below the first therapy assembly such that at least a portion of the bathtub extends within the interior chamber.

7. The therapy system of Claim 5 or Claim 6, wherein the bathtub is positioned entirely within the interior chamber of the first therapy assembly, optionally further comprising a support structure positioned adjacent to the bathtub and entirely within the interior chamber, the support structure configured to support a user within the interior chamber.

8. The therapy system of any one of the preceding Claims, wherein the interior chamber is a first interior chamber, and wherein the first therapy assembly includes a second interior chamber separated from the first interior chamber by a partition and/or further comprising one or more processing circuits configured to control at least one of a temperature of the interior chamber, a humidity of the interior chamber, or a temperature of the fluid received by the bathing volume.

9. A therapy system comprising:
a frame;
a platform supported by the frame and configured to support a user;
a first spray assembly positioned vertically above the platform, the first spray assembly including a plurality of first spray heads configured to direct a flow of fluid in a direction towards the platform; and
a second spray assembly positioned vertically below the platform, the second spray assembly including a plurality of second spray heads configured to direct a flow of fluid in a direction towards the platform.

10. The therapy system of Claim 9, further comprising a third spray assembly positioned at a lateral end of the platform, the third spray assembly including:
a third spray head configured to direct a flow of fluid in a direction towards the platform; and
an arm assembly coupled with the third spray head and configured to actuate the third spray head to selectively adjust a direction of the flow of fluid therefrom.

11. The therapy system of Claim 10, wherein the lateral end is a first lateral end and the arm assembly is a first arm assembly, the therapy system further comprising a fourth spray assembly positioned at a second lateral end of the platform opposite the first lateral end, the fourth spray assembly including:
a fourth spray head configured to direct a flow of fluid in a direction towards the platform; and
a second arm assembly coupled with the fourth spray head and configured to actuate the fourth spray head to selectively adjust a direction of the flow of fluid therefrom.

12. The therapy system of any one of Claims 9 to 11, wherein: the first spray assembly and the second spray assembly are supported by the frame; and/or the plurality of first spray heads and the plurality of second spray heads are configured to direct the flow of fluid towards the platform along a length thereof; and/or the platform includes a plurality of openings configured to permit the flow of fluid through the platform; and/or the plurality of first spray heads and the plurality of second spray heads are each movable relative to the platform to adjust a direction of the flow of fluid therefrom.

13. A therapy system comprising:
a hot therapy assembly configured to provide a hot therapy experience, the hot therapy assembly including:
a plurality of walls defining an interior chamber; and
a sauna element fluidly coupled with the interior chamber and configured to provide at least one of heated air or steam to the interior chamber; and
a cold therapy assembly configured to provide a cold therapy experience, the cold therapy assembly including a bathtub defining a bathing volume structured to receive and hold a fluid,
wherein the bathtub is positioned below the hot therapy assembly such that at least a portion of the bathtub extends within the interior chamber and the bathing volume is accessible from the interior chamber of the hot therapy assembly.

14. The therapy system of Claim 13, further comprising a support structure configured to provide selective access to the bathing volume and support a user within the interior chamber, wherein the support structure is configured to transition between a covered configuration and an uncovered configuration, wherein, in the covered configuration, access to the bathing volume is inhibited, and, wherein, in the uncovered configuration, access to the bathing volume is permitted.
